# EUROPÄISCHE PATENTSCHRIFT

(11) **EP 2 177 622 B1**
(45) Veröffentlichungstag und Bekanntmachung des Hinweises auf die Patenterteilung: **16.01.2013**
(21) Anmeldenummer: 09015830.4
(22) Anmeldetag: 12.12.2005
(51) Int. Cl.: C12Q 1/25, G01N 1/34, G01N 33/52

(54) **Chromogene Tests in Gegenwart von Zellen**
Chromogenic test in the presence of cells
Des tests chromogènes en présence des cellules

(43) Veröffentlichungstag der Anmeldung: 21.04.2010
(62) Teilanmeldung aus: 05027062.8
(73) Patentinhaber: Roche Diagnostics GmbH, 68305 Mannheim (DE); F. Hoffmann-La Roche AG, 4070 Basel (CH)
(72) Erfinder: Watzele, Manfred, 82362 Weilheim (DE); Nikolaus, Thomas, 81379 München (DE); Rode, Hans-Jürgen, 69207 Sandhausen (DE)

(56) Entgegenhaltungen:
- US-A- 4 282 001
- SAITO H ET AL: "Cytotoxicity of chlorophenols to goldfish GFS cells with the MTT and LDH assays" TOXICOLOGY IN VITRO, ELSEVIER SCIENCE, GB, Bd. 8, Nr. 5, 1. Oktober 1994 (1994-10-01) , Seiten 1107-1112, XP025543349 ISSN: 0887-2333 [gefunden am 1994-10-01]
- Roche Applied Science: "Cytotoxocity Detection Kit (LDH)" 1. Juli 2005 (2005-07-01), XP002570532 Gefunden im Internet: URL:http://www.roche-applied-science.com/p ack-insert/1644793a.pdf> [gefunden am 2010-02-24]
- VIHOLA H ET AL: "Cytotoxicity of thermosensitive polymers poly(N-isopropylacrylamide), poly(N-vinylcaprolactam) and amphiphilically modified poly(N-vinylcaprolactam)" BIOMATERIALS, ELSEVIER SCIENCE PUBLISHERS BV., BARKING, GB, Bd. 26, Nr. 16, 1. Juni 2005 (2005-06-01), Seiten 3055-3064, XP025280657 ISSN: 0142-9612 [gefunden am 2005-06-01]
- SASAKI T ET AL: "Detergent cytotoxicity: simplified assay of cytolysis by measuring LDH activity" TOXICOLOGY IN VITRO, ELSEVIER SCIENCE, GB, Bd. 6, Nr. 5, 1. September 1992 (1992-09-01), Seiten 451-457, XP025491178 ISSN: 0887-2333 [gefunden am 1992-09-01]

## Beschreibung

Die vorliegende Erfindung entstammt dem Gebiet der colorimetrischen Messung von Enzymaktivitäten in zellulären Suspensionen oder Lysaten. Insbesondere betrifft die vorliegende Erfindung ein Verfahren zur Messung von Enzymaktivitäten, bei denen die durch Zellbestandteile verursachte Trübung der Probe durch Zugabe geeigneter Additive minimiert wird.

### Stand der Technik

Will man die Wirkung von Reagenzien, Wirkstoffen, oder anderer Einflüsse auf einen Organismus prüfen, so werden diese Experimente oft in in vitro Modellen in der Zellkultur durchgeführt. Die verschiedenen enzymatischen Aktivitäten der Zellen geben dabei Aufschluss über deren Zustand und physiologische Eigenschaften.

Häufig versucht man die Aktivierung von Genen durch sogenannte Reportergen-Tests nachzuweisen. Dabei wird das Gen für ein bestimmtes Nachweisenzym (=Reportergen) hinter einen Promoter gesetzt, dessen Aktivierung man nachweisen will. Als Reportergene können hierbei auch sezernierte Enzyme wie beispielsweise die sekretorische Alkalische Phosphatase eingesetzt werden, deren Aktivität man dann mit dem Enzymsubstrat 4-Nitrophenylphosphat testet. Dieses Substrat wird dabei von diesem Enzym zum Produkt 4-Nitrophenol umgesetzt, welches bei der Wellenlänge von 405 nm Licht absorbiert.

Will man Zellen genetisch verändern, so muss man die entsprechende Plasmid-DNA mit Hilfe geeigneter Verfahren zunächst durch die Plasmamembran und dann in den Zellkern befördern. Die dabei angewendeten Verfahren wie beispielsweise die Lipofektion müssen dabei jeweils für die entsprechende Zellinie optimiert werden. Auch in diesen Fällen, wo man die Transfizierbarkeit von unterschiedlichen Zellen optimieren will, werden häufig Plasmide mit Reportergenen wie beispielsweise der sekretorischen alkalischen Phosphatase eingesetzt. Man kann dann an der gemessenen Enzymaktivität den Erfolg der Transfektion überprüfen.

Sehr häufig werden auch unterschiedliche Methoden zur Messung von zytotoxischen Eigenschaften eines Stoffes angewendet. Eine Reihe von Tests zur Zytotoxizitätsbestimmung wird in der Literatur gefunden.

Viele Tests hierzu basieren auf der Eigenschaft zytotoxischer Stoffe die Zellmembran zu schädigen. Dabei werden von Zellen deren Plasmamembran geschädigt wurde Enzyme freigesetzt, welche im Zellkulturüberstand über einen enzymatischen Test nachgewiesen werden können. Die Menge der freigesetzten Enzyme ist dabei proportional zur Anzahl der geschädigten Zellen. Derartige Enzym-Freisetzungstests wurden für die Glutamat-Oxalacetat Transaminase, für die Glutamat Pyruvat Transaminase, für die Arginosuccinat Lyase und für alkalische und saure Phosphatase beschrieben (Masanet, J., Gomez-Lechon, M. J., und Castell, J. V., Toxic. in Vitro 2 (1988) 275-282; Martin, Angela, und Clynes, Martin, In Vitro Cell Dev. Biol. 27A (1991) 183-184). Auf diese Weise wurde beispielsweise die von Lymphozyten hervorgerufene Freisetzung alkalischer Phosphatase aus humanen embryonischen Fibroblasten getestet (Szekeres, Julia, Pacsa, A.S., und Pejtsik, B., J. Immun. Meth. 40 (1981) 151-154). Am häufigsten jedoch wird für die Enzym-Freisetzungstests die Lactatdehydrogenase eingesetzt, da die Bestimmung der vorher genannten Enzyme oft durch deren geringe Menge in vielen Zellen erschwert wird. Im Gegensatz zu den anderen Enzymen ist die Lactatdehydrogenase ein sehr stabiles cytoplasmatisches Enzym, welches in allen Zellen vorhanden ist. Es wird von Zellen mit geschädigter Plasmamembran sehr schnell freigesetzt und kann leicht im Kulturüberstand nachgewiesen werden (Decker, Thomas, und Lohmann-Matthes, Marie-Luise, J. Immun. Meth. 15 (1988) 61-69; Korzeniewski, Carol, und Callewaert, Denis M., J. Immun. Meth. 64 (1983) 313-320).

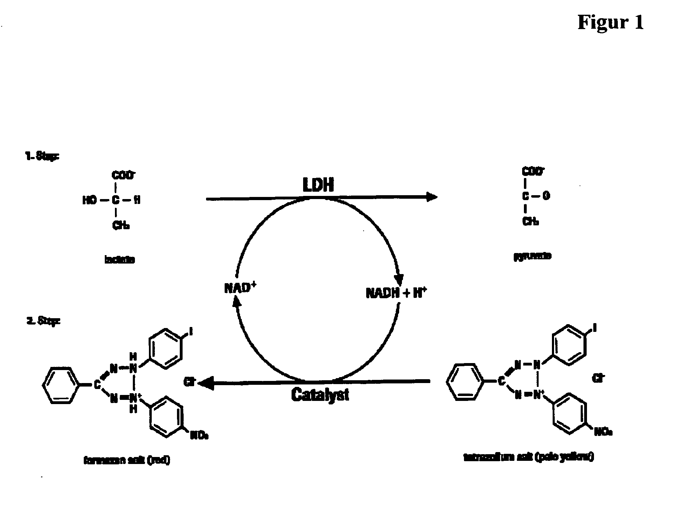

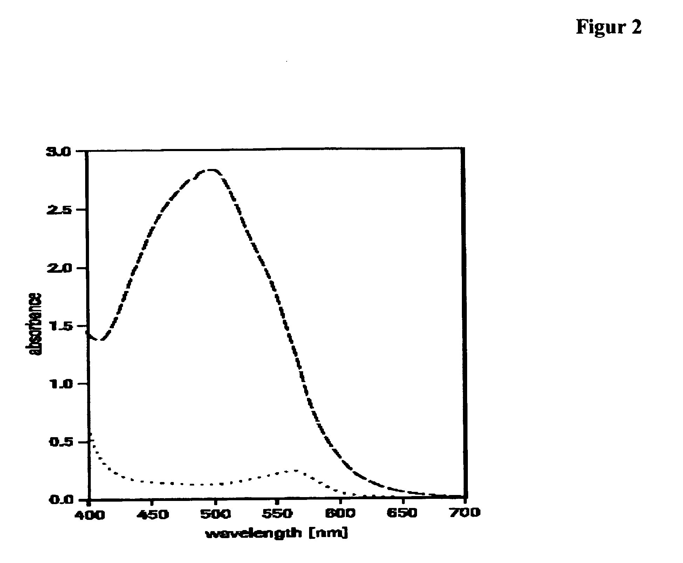

Bei diesem Test wird in einem ersten Schritt NAD+ zu NADH/H+ durch den LDHkatalysierte Umsetzung von Laktat zu Pyruvat reduziert. Im zweiten Schritt transferiert ein zweites Enzym (=Catalyst Diaphorase) H/H+ von NADH/H+ zum Tetrazolium Salz INT (2-[4-iodophenyl]-3-[4-nitrophenyl]-5-phenyltetrazolium chloride) welches dabei zum Formazan reduziert wird (Fig.1). Der Formazanfarbstoff ist wasserlöslich und zeigt ein breites Absorptionsmaximum bei 500 nm, während das Substrat INT bei dieser Wellenlänge nicht absorbiert (Fig. 2).

Bei anderen Methoden zur Zytotoxizitätsmessung werden auch in einem ähnlichen Reaktionsansatz statt colorimetrischen Enzymsubstraten fluoreszente Substrate wie Resazurin zur LDH- Messung (Cytotox-OneTM Assay der Firma Promega aus Madison, Wisconsin) oder zur Glucose-6-Phosphat Dehydrogenase Messung (Vybrant Assay der Firma Molecular Probes aus Eugene, Oregon) eingesetzt. Bei wieder anderen Methoden wird die freigesetzte Glycerinaldehyd-3-Phosphat Dehydrogenase zur Synthese von ATP über eine gekoppelte Enzymreaktion verwendet. Das dabei entstandene ATP wird in einer Biolumineszenzreaktion mit Luciferin und Luciferase eingesetzt, wobei ein messbares Lichtsignal entsteht (Corey, Michael J., et al., J. Immun. Meth. 207 (1997) 43-51).

Neben den Tests zur Messung einer zerstörten Plasmamembran wird häufig auch die physiologische Aktivität und Proliferationsrate der Zellen über die Fähigkeit bestimmte Farbsubstrate zu reduzieren ermittelt. Dabei werden Farbsubstrate eingesetzt, welche bei der Reduktion die Farbe in einer Weise ändert, dass nur das Produkt das Licht einer bestimmten Wellenlänge absorbiert, bei welcher dann gemessen wird. Bekannte Reagenzien hierfür sind beispielsweise Tetrazoliumsalze wie beispielsweise MTT (3-(4,5-dimethylthiazol-2-yl)-2,5-diphenyltetrazolium bromid), XTT (Natrium 3'-[(1-phenylamino-carbonyl)-3,4-tetrazolium]-bis(4-methoxy-6-nitro)-benzylsulfonsäure hydrat) und WST-1, welche von Roche Applied Science (Mannheim, Deutschland) unter den Namen Cell Proliferation Kit I (MTT), Cell Proliferation Kit II (XTT) und Cell Proliferation Reagent WST-1 angeboten werden.

Neben den Tetrazoliumsalzen wurde auch der blaue Farbstoff Resazurin verwendet, welcher sich bei der Reduktion in den roten, stark fluoreszenten Farbstoff Resorufin umsetzt, welcher sowohl colorimetrisch als auch fluorimetrisch gemessen werden kann (Lancaster et al., US Patent Nr. US 5,501,959).

WO 2003/089635 beschreibt eine Reagenzien-Kombination zur Bestimmung freigesetzter LDH mit den Substraten Resazurin, bzw. MTS (3-(4,5-dimethylthiazol-2-yl)-5-(3-carboxymethoxyphenyl)-2-(4-sulphophenyl)-2H-tetrazolium, inneres Salz). Dabei wird die reduzierte Form von MTS colorimetrisch und die reduzierte Form von Resazurin colorimetrisch oder bevorzugt fluorimetrisch gemessen. Bei dieser Reagenzienkombination wird auch eine Zugabe eines Stoppreagenzes vorgeschlagen, bei der die Reaktion abgestoppt wird. Vorgeschlagen als Stoppreagenz sind eine Seife, bzw. ein Detergenz oder eine starke Base wie beispielsweise NaOH. Als Seife, bzw. Detergenz wird eine 3 % SDS Lösung empfohlen. Dem Fachmann sind die Protein-denaturierenden Eigenschaften von SDS durchaus bekannt, wobei ein Stopp der Reaktion durch die Denaturierung der LDH eintritt. NaOH führt einen Reaktionsstopp durch die Änderungen des pH-Wertes herbei. Allerdings führen die wenigsten bekannten Detergenzien, oder Seifen zu einem Reaktionsstopp, wie die unten angegebenen Beispiele belegen. Die Lehre in WO 2003/089635 wäre also solche Detergenzien oder Seifen zu verwenden, welche einen Reaktionsstopp bewirken würden, also Protein-denaturierende Eigenschaften haben. Die in diesem Patent angegebenen Seifen oder Detergenzien enthalten zudem keinen Hinweis darauf, dass durch deren Verwendung eine Verringerung der Lichtstreuung oder Absorption verursacht durch Zellen vermieden und dadurch eine Erhöhung der Sensitivität colorimetrischer Messungen erreicht werden kann. Vihola et al. (Biomaterials, Bd. 26 Nr. 1, p. 3055-3064, 2005) beschreibt ein Verfahren, bei dem zu Beginn INT zugegeben wird, nach 30 Minuten mit Salzsäure gestoppt wird und die colorimetrische Messung ohne vorherige Zentrifugation erfolgt.

Bei allen Methoden, bei welchen eine Enzymaktivität in Gegenwart von Zellen colorimetrisch detektiert wird, gibt es insbesondere bei hohen Zellzahlen das Problem, dass das Licht im gesamten Wellenlängenbereich des sichtbaren Lichtes von den Zellen gestreut und absorbiert wird. So wird beispielsweise in der von Decker und Lohmann-Matthes beschriebenen Methode zur Messung von freigesetzter Lactatdehydrogenase darauf hingewiesen, dass man bei hohen Zellzahlen wegen der dabei beobachteten hohen Absorption der Zellen besser die zellfreien Überstände aus der Zellkulturplatte in neue Reaktionsgefäße überführt und dort die Messung der enzymatischen Aktivität durchführt. Dieses Vorgehen erfordert eine Zentrifugation der Kulturplatten und ein vorsichtiges Entfernen des Kulturüberstandes. Dies sind zusätzliche Arbeitsschritte, die eine Anwendung in der Hochdurchsatzanalyse beispielsweise mit entsprechenden Robotern erschweren. Zusätzlich können durch das Zentrifugieren oder das Abnehmen des Überstandes die Zellen beschädigt werden und dabei cytoplasmatische Enzyme freigesetzt werden, wodurch das Testergebnis gestört werden kann.

Bei den anderen Verfahren, bei denen eine fluorimetrische oder eine chemilumineszente Messung erfolgt kann man nicht mit den insbesondere für Standard Mikrotiterplatten weit verbreiteten colorimetrischen Messgeräten, den sogenannten ELISA Readern arbeiten, was die Nutzung dieser Substrate erheblich einschränkt. Bei den chemilumineszenten Substraten ist häufig eine relativ kurze Stabilität des Signals gegeben, was eine rasche Messung unmittelbar nach Ende des Testes erfordert.

In der in WO 2003/089635 veröffentlichten Methode zur Bestimmung freigesetzter LDH mit den Substraten Resazurin, bzw. MTS, werden diese auch für colorimetrische Messungen beschrieben. Als Stoppreagenz wird hierbei NaOH oder SDS beansprucht. Hierbei muss jedoch beachtet werden, dass die colorimetrische Messung des reduzierten Resazurin nur eine geringe Sensitivität erlaubt. Die Verwendung von NaOH oder SDS bei Tetrazoliumsalzen wie beispielsweise MTS ist von Nachteil, da NaOH zu einer Präzipitation von reduzierten Tetrazoliumsalzen führt (siehe Beispiel 2 dieser Anmeldung). Auch SDS ist nicht bevorzugt, da hierbei Tetrazoliumsalze überraschenderweise relativ schnell ausbleichen (siehe Beispiel 2 dieser Anmeldung).

### Kurzbeschreibung der Erfindung

Gegenstand der vorliegenden Erfindung ist deshalb ein Verfahren zur colorimetrischen Messung von Enzymaktivitäten, enthaltend folgende Schritte:
- Bereitstellen einer Zellsuspension oder eines Zellbestandteile enthaltenden Zell-Lysates.
- Zugabe von INT (2-[4-Iodophenyl]-3-[4-nitrophenyl]-5-phenyltetrazoliumchlorid) zu Beginn der Enzymreaktion,
- Zugabe von HCL (Salzsäure) als einer ersten weiteren Substanz zum Abstoppen der Enzymreaktion,
- Zugabe einer zweiten weiteren Substanz, welche entweder Cetyltrimethylammonium Bromid oder Dodecyltrimethylammonium Bromid ist,
   nach Beendigung der Enzymreaktion, die Zellmembranen auflöst,
- colorimetrische Messung ohne vorherige Zentrifugation.

### Kurzbeschreibung der Abbildungen

### Figur 1

Im ersten Schritt reduziert ins Medium freigesetzte Lactat Dehydrogenase (LDH) NAD+ zu NADH+ H+ durch Oxidation von Lactat zu Pyruvat. In der zweiten enzymatischen Reaktion werden 2 H von NADH+ H+ auf das Tetrazolium Salz INT (2-[4-iodophenyl]-3-[4-nitrophenyl]-5-phenyltetrazolium chlorid) übertragen.

### Figur 2

Absorptionsspektrum der Working Solution aus dem Cytotoxicity Detection Kit (LDH). Die Reaction Mixture aus dem Cytotoxicity Detection Kit (LDH) wurde zu RPMI 1640 Medium mit 1 % Rinderserumalbumin (BSA) gegeben und das Absorptionsspektrum in Abwesenheit (.......) und Anwesenheit (——) von LDH gemessen.

### Detailierte Beschreibung der Erfindung

Die vorliegende Erfindung beruht auf einem Verfahren zur colorimetrischen Messung von Enzymaktivitäten in Gegenwart von Zellen, gekennzeichnet dadurch, dass zu den Zellen ein oder mehrere Substanzen zugesetzt werden, welche die durch die Zellen verursachte Lichtstreuung und Absorption minimieren.

Dies wird im Wesentlichen erreicht durch ein Verfahren, enthaltend die folgende Schritte
- Bereitstellen einer Zellsuspension oder eines Zellbestandteile enthaltenden Zell-Lysates.
- Zugabe von INT (2-[4-Iodophenyl]-3-[4-nitrophenyl]-5-phenyltetrazoliumchlorid) zu Beginn der Enzymreaktion,
- Zugabe von HCL (Salzsäure) als einer ersten weiteren Substanz zum Abstoppen der Enzymreaktion,
- Zugabe einer zweiten weiteren Substanz, welche entweder Cetyltrimethylammonium Bromid oder Dodecyltrimethylammonium Bromid ist,
   nach Beendigung der Enzymreaktion, die Zellmembranen auflöst,
- colorimetrische Messung ohne vorherige Zentrifugation.

Üblicherweise muss bei hohen Zelldichten erst ein zellfreier Überstand durch Zentrifugation gebildet werden der dann nach dessen Abnahme vermessen werden kann. Die hier beschriebene Erfindung vermeidet diese zusätzlichen Arbeitsschritte dadurch, dass zu den Zellen am Ende des Testes, Reagenzien zugesetzt werden, welche die durch die Zellen verursachte Lichtstreuung und Absorption minimieren.

Bevorzugt geeignet zur Permeabilisierung sind Detergenzien. Dem Fachmann sind eine Vielfalt unterschiedlicher Detergenzien bekannt. Unterschieden wird hierbei zwischen ionischen, nicht-ionischen und zwitterionischen Detergenzien. Innerhalb der ionischen Detergenzien wird weiter zwischen anionischen Detergenzien wie beispielsweise SDS (Natriumdodecylsulfat), N-Laurylsarcosin oder Natriumcholat und kationischen Detergenzien wie beispielsweise Cetyltrimethylammonnium Bromid (CTAB), oder Dodecyltrimethylamonnium Bromid (DTAB) unterschieden. Beispiele für nicht-ionische Detergenzien sind dem Fachmann unter den Namen Triton®X-100 (Octylphenoxypolyethoxyethanol), Nonidet P40 oder TWEEN® 20 (Polyoxyethylen(20)sorbitan Monolaurat) bekannt. Gebräuchliche Zwitterionische Reagenzien sind beispielsweise CHAPS ([3-(3-Cholamidopropyl) dimethylammonio]-1-propansulfonsäure) oder Zwittergent®3-12 (n-Dodecyl-N,N dimethyl-3-ammonio-1-propansulfonsäure).

Überraschenderweise konnte mit den kationischen Detergenzien CTAB und DTAB eine Erhöhung der Absorption des reduzierten INT Substrates erreicht werden, was zu einer Sensitivitätssteigerung im Test führte. Zusätzlich waren die Signale mit diesen Detergenzien über einen Zeitraum von 5 Tagen sehr stabil, während mit reinem PBS-Puffer oder mit dem nicht-ionischen Detergenz TritonX100 eine deutliche Signalabnahme erfolgte und das anionische Detergenz SDS einen raschen Verlust der Absorption bewirkten.

CTAB oder DTAB werden erfindungsgemäß bis zu einer Endkonzentration bis maximal 3 % (v/v) und vorzugsweise bis maximal 1 % (v/v) zugesetzt.

Wird zur Erniedrigung der Lichtstreuung und Absorption der Zellen eine Permeabilisierung der Zellmembran durchgeführt, ist darauf zu achten, dass durch diesen Schritt freigesetzte Stoffe nicht die Reaktion beeinflussen. So könnten beispielsweise Enzyme freigesetzt werden, die weiteres Reaktionsprodukt herstellen, oder das Reaktionsprodukt weiter umsetzen, sodass es nicht mehr messbar ist. Es ist daher für einige enzymatische Reaktionen sinnvoll, gleichzeitig mit der Permeabilisierung der Zellen einen Stopp der Reaktion durchzuführen. Geeignete Maßnahmen hierfür sind abhängig von der zu messenden Reaktion.

Geeignete Massnahmen, um eine enzymatische Reaktion abzustoppen kann beispielsweise die Zugabe spezifischer Enzyminhibitoren, oder die Änderung der Reaktionsbedingungen in der Art sein, sodass die Reaktion beendet wird. In vielen Fällen ist die Änderung des pH-Wertes im Reaktionsmedium geeignet einen Reaktionsstopp zu bewirken. In den hier in den Beispielen untersuchten Fällen wurden NaOH und bevorzugt HCl zur Änderung des pH Wertes eingesetzt. HCl wurde auch im Cytotoxicity Detection Kit (LDH) (Roche Applied Science Cat. No. 11644793001) als Stoppreagenz für die LDH vorgeschlagen. Decker und Lohmann-Matthes (Decker, Thomas, und Lohmann-Matthes, Marie-Luise, J. Immun. Meth. 15 (1988) 61-69) schlagen in Ihrer Arbeit eine 15 mM Natriumoxamat Lösung zum Abstoppen von LDH als Inhibitor vor.

Darüber hinaus können die enzymatischen Reaktionen durch Zugabe von Proteindenaturierenden Stoffen wie beispielsweise Harnstoff oder durch Zugabe von Proteasen gestoppt werden.

Gegenstand der vorliegenden Erfindung ist auch die Verwendung von bestimmten Substanzen bei der colorimetrischen Messung von Enzymaktivitäten in Gegenwart von Zellen oder Zellbestandteilen, dadurch gekennzeichnet dadurch dass die verwendeten Substanzen die durch die Zellen oder Zellbestandteile verursachte Lichtstreuung und Absorption reduzieren und dabei die Enzymaktivitäten nicht beeinflusst werden.

Bevorzugt ist die Verwendung von Cetyltrimethylammonnium Bromid (CTAB) oder Dodecyltrimethylamonnium Bromid (DTAB).

### Beispiele

### Beispiel 1

### Messung der Absorption von Zellen nach Behandlung mit unterschiedlichen Reagenzien

Die humane Suspensionszelllinie U 937 wurde in RPMI 1640 Medium (Firma Sigma) mit 10 % fötalem Kälberserum und 2 mM Glutamine angezüchtet. Die Zellen wurden dann in frischem RPMI Medium gewaschen und dabei auf 25 000, 50 000, bzw. 100 000 Zellen/100 µl eingestellt. Davon wurde jeweils 100 µl in eine 96 Loch Standard Mikrotiterplatte übertragen. Zu den Zelllösungen wurde jeweils 50 µl RPMI Medium mit den entsprechenden Reagenzien pipettiert.

Nach 5 min und 75 min Inkubation wurde jeweils die Absorption bei 690 nm Wellenlänge gemessen.

In der folgenden Tabelle erkennt man, dass die Absorption der Zellen mit zunehmender Zellzahl steigt. Außerdem wird deutlich, dass alle Reagenzien die Absorption gegenüber dem reinen Medium senken, wobei die optimalen Einsatzkonzentrationen noch zu ermitteln sind.

| Reagenz | Zellzahl | Absorption nach 5 min | Absorption nach 75 min |
|---|---|---|---|
| Triton X100 ad 1 % (V/V) | 25 000 Zellen | 0,001 | 0,001 |
| | 50 000 Zellen | 0,004 | 0,004 |
| | 100 000 Zellen | 0,008 | 0,008 |
| SDS ad 1 % (VN) | 25 000 Zellen | 0,002 | 0,003 |
| (Stand der Technik) | 50 000 Zellen | 0,006 | 0,006 |
| | 100 000 Zellen | 0,010 | 0,010 |
| CTAB ad 1 % (V/V) | 25 000 Zellen | 0,006 | 0,003 |
| | 50 000 Zellen | 0,031 | 0,014 |
| | 100 000 Zellen | 0,025 | 0,018 |
| DTAB ad 1 % (V/V) | 25 000 Zellen | 0,005 | 0,006 |
| | 50 000 Zellen | 0,012 | 0,011 |
| | 100 000 Zellen | 0,024 | 0,021 |
| N-Laurylsarcosin ad | 25 000 Zellen | 0,001 | 0,002 |
| 1 % (V/V) | 50 000 Zellen | 0,003 | 0,004 |
| | 100 000 Zellen | 0,012 | 0,013 |
| RPMI | 25 000 Zellen | 0,01 | 0,011 |
| (negative Kontrolle) | 50 000 Zellen | 0,018 | 0,018 |
| | 100 000 Zellen | 0,044 | 0,048 |

### Beispiel 2

### Abstoppen der LDH Reaktion mit verschiedenen Stoppreagenzien.

Für diesen Versuch werden zunächst Lysate von U 937 Zellen als Quelle für LDH hergestellt. Dazu wurden 6x105 Zellen in 1 ml destilliertem Wasser suspendiert und mit Ultraschall behandelt, bis alle Zellen lysiert waren. Anschließend wurde das Lysat für 10 min. bei 200 xg zentrifugiert und der Überstand frei von Zellresten für weitere Versuche verwendet.

Zellfreie Lysate von je 5000 Zellen wurde in einen LDH Test mit Komponenten aus dem Cytotoxicity Detection Kit (LDH) (Roche Applied Science Cat. No: 11644793) eingesetzt.

Abweichend von dem erwähnten Kit wurde eine Lösung bestehend aus 2 mM INT, 90 mM L-Lactat, 100 mM Tris, pH 8,5 ohne Detergenz als "Dye Solution" eingesetzt.

Nach einer Reaktionszeit von 5 min wurden zu diesen Ansätzen unterschiedliche Stoppreagenzien zugesetzt und die Absorption bei 492 nm (dem Absorptionsmaximum von reduzierten INT) verfolgt.

| Stoppreagenz | Absorption 0 min nach Stoppreagenz Zugabe | Absorption 30 min nach Stoppreagenz Zugabe | Absorption 120 min nach Stoppreagenz Zugabe |
|---|---|---|---|
| RPMI Medium (negative Kontrolle) | 0,113 | 0,309 | 0,852 |
| SDS ad 3 % (V/V) (Stand der Technik) | 0,113 | 0,113 | 0,105 |
| 15 mM Natriumoxamat | 0,113 | 0,127 | 0,179 |
| 15 mM Natriumoxamat + Triton X100 ad 1 % (V/V) | 0,113 | 0,127 | 0,191 |

In der Tabelle erkennt man, dass sowohl SDS, als auch Natriumoxamat die LDH Reaktion stoppen. Allerdings gelingt der Stopp mit der angewendeten Oxamat-Konzentration nicht zu 100 %, wie das bei SDS der Fall ist. Eine Optimierung der Oxamat-Konzentration wäre hier nötig. Das Beispiel mit der Kombination von Oxamat mit Triton X100 zeigt, dass dieser Inhibitor auch in Gegenwart dieses Detergenzes aktiv ist. Bei der Verwendung von SDS ist eine leichte Abnahme der Absorption zu beobachten, was auf eine Instabilität des Substrates in Kombination mit SDS deutet.

In einem weiteren Versuch wurde HCl und NaOH als Stoppreagenz eingesetzt

Hierfür wurden zellfreie Lysate von je 1000 Zellen in einen LDH Test mit Komponenten aus dem Cytotoxicity Detection Kit (LDH) mit der modifizierten "Dye Solution" eingesetzt. Nach einer Reaktionszeit von 30 min wurden zu diesen Ansätzen unterschiedliche Stoppreagenzien zugesetzt und die Absorption bei 492 nm (dem Absorptionsmaximum von reduzierten INT) verfolgt.

| Stoppreagenz | Absorption 0 min nach Stoppreagenz Zugabe | Absorption 30 min nach Stoppreagenz Zugabe | Absorption 120 min nach Stoppreagenz Zugabe |
|---|---|---|---|
| RPMI Medium | 0,280 | 0,448 | 0,962 |
| 0,2 M HCl | 0,275 | 0,271 | 0,261 |
| 0,2 M HCl + Triton X100 ad 1 % (V/V) | 0,268 | 0,269 | 0,261 |

Hier wird erkennbar, dass HCl ein geeignetes Stoppreagenz für die LDH Reaktion darstellt. Die Kombination von HCl mit Triton X100 führt hier zum gleichen Ergebnis.

Die Zugabe von NaOH in 0,2 M Endkonzentration führte zur sofortigen Präzipitation des Farbstoffes.

### Beispiel 3

### Messung unterschiedlicher Mengen freigesetzter LDH in Gegenwart hoher Zellmengen

Zellfreie Lysate von 50 000, 10 000 und 1000 U 937 Zellen wurden in 50 µl Volumen in den Vertiefungen einer Standardmikrotiterplatte für 0, 5, bzw. 30 Minuten in einem LDH Test mit 100 µl Reaktionslösung aus dem Cytotoxicity Detection Kit (LDH) mit modifizierter "Dye Solution" bei Raumtemperatur inkubiert. Nach 5, bzw. 30 Minuten Inkubationszeit wurde mit 6.5 µl HCl 25 %-ig die Reaktion abgestoppt. Anschließend wurden zu diesen Ansätzen jeweils 50 µl mit 150 000 U937 Zellen und gleichzeitig 50 µl verschiedener Detergenzienlösungen zugegeben.

Anschließend wurde bei einer Wellenlänge von 492 nm jeweils nach 10 Minuten und nach 5 Tagen gemessen. Die Leerwerte eines Kontrollansatzes, bei welchem die Reaktion sofort nach 0 Minuten abgestoppt wurden, wurden jeweils von den Messwerten subtrahiert.

| Reagenz | Lysatmenge (Zellzahl) | Absorption nach 5 min Messung 10 min nach Abstoppen der Reaktion (Messung nach 5 Tagen) | | Absorption nach 30 min Messung 10 min nach Abstoppen der Reaktion (Messung nach 5 Tagen) | |
|---|---|---|---|---|---|
| PBS (Phosphatpuffer mit Salzen) (negative Kontrolle) | 50 000 Zellen | 0,048 | (0,024) | 0,288 | (0,119) |
| | 10 000 Zellen | 0,000 | (0,000) | 0,067 | (0,033) |
| | 1 000 Zellen | 0,000 | (0,000) | 0,000 | (0,000) |
| | 0 Zellen | 0,000 | (0,000) | 0,000 | (0,000) |
| Triton X100 ad 1 % (V/V) | 50 000 Zellen | 0,101 | (0,093) | 0,338 | (0,150) |
| | 10 000 Zellen | 0,038 | (0,035) | 0,078 | (0,065) |
| | 1 000 Zellen | 0,003 | (0,001) | 0,006 | (0,001) |
| | 0 Zellen | 0,000 | (0,000) | 0,000 | (0,000) |
| SDS ad 1 % (V/V) (Stand der Technik) | 50 000 Zellen | 0,093 | (0,008) | 0,364 | (0,021) |
| | 10 000 Zellen | 0,011 | (0,000) | 0,078 | (0,000) |
| | 1 000 Zellen | 0,000 | (0,000) | 0,007 | (0,000) |
| | 0 Zellen | 0,000 | (0,000) | 0,000 | (0,000) |
| CTAB ad 1 % (V/V) | 50 000 Zellen | 0,095 | (0,085) | 0,372 | (0,363) |
| | 10 000 Zellen | 0,036 | (0,021) | 0,084 | (0,077) |
| | 1 000 Zellen | 0,001 | (0,002) | 0,010 | (0,004) |
| | 0 Zellen | 0,000 | (0,000) | 0,000 | (0,000) |
| DTAB ad 1 % (V/V) | 50 000 Zellen | 0,088 | (0,082) | 0,370 | (0,299) |
| | 10 000 Zellen | 0,034 | (0,026) | 0,075 | (0,069) |
| | 1 000 Zellen | 0,002 | (0,002) | 0,008 | (0,005) |
| | 0 Zellen | 0,000 | (0,000) | 0,000 | (0,000) |

Man erkennt, dass man durch die Zugabe der Detergenzien die LDH aus dem Lysat von 10000 Zellen bereits nach 5 min Reaktionszeit in Gegenwart von 150 000 ganzen Zellen nachweisen kann, während dies bei Verwendung reinen PBS Puffers ohne Detergenz nicht möglich ist. Nach 30 min Reaktionszeit kann bereits die LDH aus 1000 Zellen in diesem Hintergrund in den Detergenz-behandelten Proben nachgewiesen werden, während ohne Detergenz nur 10000 Zellen detektierbar sind. Dies liegt daran, dass bei den nicht Detergenz-behandelten Proben eine höhere Hintergrund-Absorption verursacht durch die 150 000 Zellen vorhanden ist.

Überraschenderweise kann man feststellen, dass für dieses Farbsubstrat kationische Detergenzien wie CTAB und DTAB die Messwerte über 5 Tage nahezu stabil halten, während SDS hier destabilisierend wirkt.

## Patentansprüche

1. Verfahren zur colorimetrischen Messung einer Lactatdehydrogenase (LDH) Reaktion, enthaltend folgende Schritte:
- Bereitstellen einer Zellsuspension oder eines Zellbestandteile enthaltenden Zell-Lysates,
- Zugabe von INT (2-[4-Iodophenyl]-3-[4-nitrophenyl]-5-phenyltetrazoliumchlorid) zu Beginn der Enzymreaktion,
- Zugabe von HCL (Salzsäure) als einer ersten weiteren Substanz zum Abstoppen der Enzymreaktion,
- Zugabe einer zweiten weiteren Substanz, welche entweder Cetyltrimethylammonium Bromid oder Dodecyltrimethylammonium Bromid ist,
nach Beendigung der Enzymreaktion, die Zellmembranen auflöst,
- colorimetrische Messung ohne vorherige Zentrifugation.

## Claims

1. Method for the colorimetric measurement of a lactate dehydrogenase (LDH) reaction comprising the following steps:
- providing a cell suspension or a cell lysate containing cell components,
- adding INT (2-[4-iodophenyl]-3-[4-nitrophenyl]-5-phenyltetrazolium chloride) at the start of the enzyme reaction,
- adding HCl (hydrochloric acid) as a first additional substance to stop the enzyme reaction,
- adding a second additional substance which is either cetyltrimethylammonium bromide or dodecyltrimethylammonium bromide after completion of the enzyme reaction which dissolves cell membranes,
- colorimetric measurement without prior centrifugation.

## Revendications

1. Procédé pour la mesure colorimétrique d'une réaction de lactate déshydrogénase (LDH), contenant les étapes suivantes dans lesquelles :
- on procure une suspension cellulaire ou bien un lysat cellulaire contenant des constituants cellulaires ;
- on ajoute du INT (chlorure de 2-[4-iodophényl]-3-[4-nitrophényl]-5-phényltétrazolium) au début de la réaction enzymatique ;
- on ajoute du HCl (acide chlorhydrique) à titre d'une première substance supplémentaire pour l'arrêt de la réaction enzymatique ;
- on ajoute une deuxième substance supplémentaire qui représente soit du bromure de cétyltriméthylammonium, soit du bromure de dodécyltriméthylammonium, au terme de la réaction enzymatique, qui désintègre les membranes cellulaires ;
- on procède à une mesure colorimétrique en l'absence d'une centrifugation préalable.
